# FASCICULE DE BREVET EUROPEEN

(11) **EP 1 019 758 B1**
(45) Date de publication et mention de la délivrance du brevet: **06.03.2002**
(21) Numéro de dépôt: 98947621.3
(22) Date de dépôt: 07.10.1998
(51) Int. Cl.: G02B 3/14, G02B 26/02

(54) **LENTILLE A FOCALE VARIABLE**
LINSE MIT VERSTELLBARER BRENNWEITE
LENS WITH VARIABLE FOCUS

(30) Priorité: 08.10.1997 FR 9712781
(43) Date de publication de la demande: 19.07.2000
(73) Titulaire: UNIVERSITE JOSEPH FOURIER, 38041 Grenoble Cédex 9 (FR)
(72) Inventeur: BERGE, Bruno, F-38140 Renage (FR); PESEUX, Jérôme, F-25000 Besançon (FR)
(74) Mandataire: de Beaumont, Michel
(86) Numéro de dépôt international: FR9802143
(87) Numéro de publication internationale: WO9918456

(56) Documents cités:
- FR-A- 822 886
- US-A- 4 030 813
- US-A- 5 659 330
- SHERIDON N K: "ELECTROCAPILLARY IMAGING DEVICES FOR DISPLAY AND DATA STORAGE" XEROX DISCLOSURE JOURNAL, vol. 4, no. 3, mai 1979, page 385/386 XP002037058
- BERGE B: " lectrocapillarité et mouillage de films isolants par l'eau" COMPTES RENDUS DES SEANCES DE L'ACADEMIE DES SCIENCES, vol. 317, no. 2, 22 juin 1993, pages 157-163, XP002068041 PARIS cité dans la demande

## Description

La présente invention concerne le domaine des lentilles à focale variable, plus particulièrement des lentilles liquides à focale variable contrôlées électriquement.

Un article de B. Berge intitulé "Electrocapillarité et mouillage de films isolants par l'eau" publié en 1993 dans C.R. Acad. Sci. Paris, t. 317, série II, pages 157 à 163, présente un dispositif comprenant une goutte d'un liquide conducteur posée sur un film diélectrique recouvrant une électrode plane. Une tension électrique peut être appliquée entre la goutte de liquide conducteur et l'électrode. Cet article décrit une étude théorique de la variation de la mouillabilité d'un matériau diélectrique vis-à-vis d'un liquide conducteur et montre que la mouillabilité augmente sensiblement en présence d'un champ électrique dû à la tension électrique existant entre le liquide conducteur et l'électrode. Ce phénomène est appelé électromouillage par l'auteur.

Le brevet des États-Unis d'Amérique numéro 5659330 décrit un dispositif d'affichage utilisant le phénomène d'électromouillage pour faire varier la forme d'une goutte d'un liquide conducteur opaque posée sur un diélectrique. Ce document ne suggère pas d'application comme lentille optique.

Un article de Vallet, Berge et Vovelle, "Electrowetting of water and aqueous solutions on poly(ethylene terephthalate) insulating films", publié dans Polymer, Vol.37, No. 12, pages 2465 à 2470, 1996, décrit la déformation d'une goutte de liquide conducteur à laquelle est appliquée une tension. Il est indiqué que lorsque la tension appliquée devient importante, les contours de la goutte deviennent instables, et des microgouttes peuvent être expulsées à la périphérie de la goutte.

Ceci entraîne que les systèmes antérieurs ne sont pas adaptés à la formation de lentilles variables. En outre ces systèmes nécessiteraient une électrode de polarisation transparente et une connexion pour l'électrode ce qui rend le système difficile à fabriquer ou inefficace.

Un objet de la présente invention est de prévoir une lentille dont la focale peut varier continûment en fonction d'une commande électrique, en utilisant le phénomène d'électromouillage.

Un autre objet de la présente invention est de prévoir une lentille simple à fabriquer.

Un autre objet de la présente invention est de prévoir une lentille simple à mettre en oeuvre.

Pour atteindre ces objets, la présente invention prévoit une lentille à focale variable comprenant une enceinte remplie d'un premier liquide, une goutte d'un deuxième liquide étant disposée au repos sur une zone d'une première face d'une paroi isolante de l'enceinte, les premier et deuxième liquides étant non miscibles, d'indices optiques différents et sensiblement de même densité, dans laquelle le premier liquide est conducteur, le deuxième liquide est isolant, et qui comprend des moyens pour appliquer une tension électrique entre le liquide conducteur et une électrode disposée sur la deuxième face de ladite paroi et des moyens de centrage pour maintenir le centrage et contrôler la forme du bord de la goutte tandis qu'une tension est appliquée.

Selon un mode de réalisation de la présente invention, les moyens de centrage permettent de maintenir continûment le centrage de la goutte et de contrôler continûment la forme du bord de la goutte tandis qu'une tension variable est appliquée par lesdits moyens pour appliquer une tension électrique.

Selon un mode de réalisation de la présente invention, la première face est sensiblement plane, la zone de contact est circulaire et centrée sur un axe perpendiculaire à la première face.

Selon un mode de réalisation de la présente invention, les moyens de centrage correspondent à un épaississement progressif de la deuxième face de la paroi de l'enceinte vers ledit axe, ladite électrode étant plaquée sur ladite deuxième face.

Selon un mode de réalisation de la présente invention, les moyens de centrage correspondent à une décroissance radiale de la mouillabilité vis-à-vis du premier liquide, vers le centre de ladite zone de contact avec le deuxième liquide.

Selon un mode de réalisation de la présente invention, les moyens de centrage correspondent à une gradation radiale de la constante diélectrique de ladite paroi de l'enceinte au niveau de ladite zone de contact avec le deuxième liquide.

Selon un mode de réalisation de la présente invention, la première face est sensiblement plane, la zone de contact est circulaire et centrée sur un axe perpendiculaire à la première face, et les moyens de centrage sont constitués d'une électrode composée d'une ou plusieurs bandes circulaires concentriques isolées entre elles, centrées sur ledit axe, les bandes circulaires étant alimentées par des sources de tension distinctes de valeur décroissante vers ledit axe.

Selon un mode de réalisation de la présente invention, l'enceinte est cylindrique, la première face est la face intérieure de l'enceinte, la zone de contact avec le deuxième liquide correspond à une section cylindrique de l'enceinte, les moyens de centrage sont constitués d'une ou plusieurs électrodes cylindriques de même diamètre, isolées entre elles, disposées côte à côte contre la face extérieure de l'enceinte au niveau de la frontière de ladite zone de contact, les électrodes étant alimentées par des tensions différentes de valeur décroissante vers le milieu de ladite zone de contact.

Selon un mode de réalisation de la présente invention, la première face est sensiblement plane, la zone de contact est rectangulaire et symétrique par rapport à un axe perpendiculaire à la première face et les moyens de centrage sont constitués d'une électrode composée d'une ou plusieurs bandes rectangulaires concentriques isolées entre elles, symétriques par rapport audit axe, les bandes rectangulaires étant alimentées par des sources de tension distinctes de valeur décroissante vers ledit axe.

Selon un mode de réalisation de la présente invention, ladite paroi est composée de deux plans non parallèles et ladite zone se trouve à cheval sur lesdits deux plans.

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
la figure 1 représente un premier mode de réalisation d'une lentille à focale variable selon la présente invention ;
la figure 2 représente un deuxième mode de réalisation d'une lentille à focale variable selon la présente invention ;
la figure 3 représente un troisième mode de réalisation d'une lentille à focale variable selon la présente invention ;
la figure 4 représente un quatrième mode de réalisation d'une lentille à focale variable selon la présente invention ;
la figure 5 représente un cinquième mode de réalisation d'une lentille à focale variable selon la présente invention ; et
la figure 6 représente un autre mode de réalisation d'une lentille à focale variable selon la présente invention.

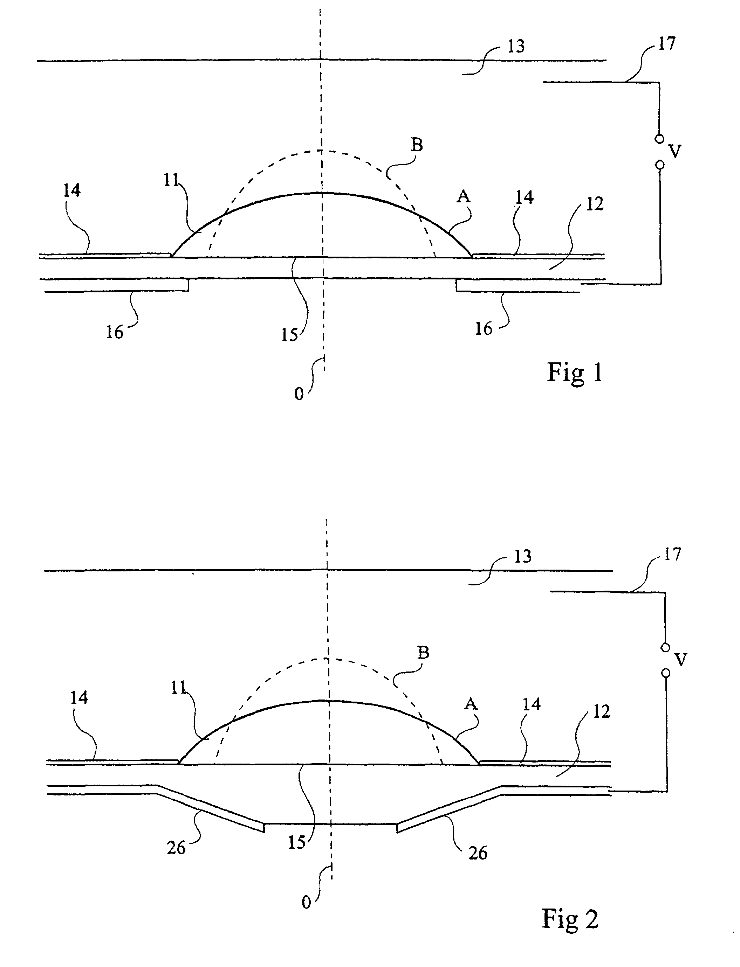

La figure 1 représente une vue en coupe simplifiée d'une lentille liquide à focale variable selon un premier mode de réalisation de la présente invention. Une goutte d'un liquide isolant 11 est posée sur la surface intérieure d'une paroi d'une enceinte diélectrique 12 remplie d'un liquide conducteur 13. Le liquide isolant 11 et le liquide conducteur 13 sont tous les deux transparents, non miscibles, sont d'indices optiques différents et ont sensiblement la même densité. Le diélectrique 12 présente naturellement une faible mouillabilité vis-à-vis du liquide conducteur 13. Un traitement de surface 14 assurant une forte mouillabilité de la paroi de l'enceinte diélectrique vis-à-vis du liquide conducteur 13 entoure la zone de contact 15 entre la goutte de liquide isolant 11 et la paroi de l'enceinte 12. Le traitement de surface 14 permet de conserver le positionnement de la goutte 11, pour éviter que le liquide isolant ne s'étale au-delà de la surface de contact souhaitée. Lorsque le système est au repos, la goutte de liquide isolant 11 prend naturellement la forme désignée par la référence A. On appelle O l'axe perpendiculaire à la zone de contact 15, passant par le centre de la zone de contact 15. Au repos, la goutte de liquide isolant 11 est centrée sur l'axe O qui constitue l'axe optique du dispositif. Les éléments du dispositif voisins de l'axe O sont transparents. Une électrode 16, laissant passer la lumière au voisinage de l'axe O, est placée sur la face extérieure de la paroi de l'enceinte diélectrique 12 sur laquelle est située la goutte de liquide isolant 11. Une électrode 17 est en contact avec le liquide conducteur 13. L'électrode 17 peut être immergée dans le liquide 13, ou bien être un dépôt conducteur réalisé sur une paroi interne de l'enceinte 12.

Lorsqu'on établit une tension V entre les électrodes 16 et 17, on crée un champ électrique qui, selon le principe d'électromouillage susmentionné, va accroître la mouillabilité de la zone 15 vis-à-vis du liquide conducteur 13. En conséquence, le liquide conducteur 13 se déplace et déforme la goutte de liquide isolant 11. On obtient ainsi une variation de la focale de la lentille.

Cependant le centre de la goutte est susceptible de se déplacer par rapport à l'axe O lors de sa déformation. En outre, le contour de la surface de contact est susceptible de perdre son caractère circulaire lors de la déformation de la goutte. Un aspect de la présente invention est de maintenir la goutte circulaire et centrée sur l'axe O lors de sa déformation en générant un champ électrique décroissant radialement vers le centre de la zone 15.

Pour éviter cela, selon un aspect de la présente invention, on prévoit en outre un moyen de centrage de la goutte 11. Des exemples d'un tel moyen de centrage apparaissent dans les deuxièmes à sixièmes modes de réalisation de l'invention décrits ci-après.

La figure 2 représente une vue en coupe simplifiée d'une lentille liquide à focale variable selon un deuxième mode de réalisation de la présente invention. Les éléments tels que la goutte 11, l'axe O, l'enceinte 12, le liquide conducteur 13, le traitement de surface 14, la zone de contact 15 et l'électrode 17 sont les mêmes que dans le mode de réalisation illustré en figure 1. Les positions A et B correspondent également à la position de repos de la goutte 11 et à la position limite de la goutte 11, respectivement. Dans ce deuxième mode de réalisation, le moyen de centrage est constitué par la génération d'un champ électrique décroissant radialement vers le centre de la zone 15. Pour cela, on prévoit une électrode 26 qui a une surface qui s'éloigne progressivement de la surface de la zone 15 à mesure que l'on se rapproche de l'axe O. On peut obtenir une telle électrode 26 par exemple en déposant une pellicule métallique sur les parois latérales d'un tronc de cône centré sur l'axe O, réalisé sur la paroi extérieure de la face de l'enceinte 12 sur laquelle est placée la goutte 11. Une variante de réalisation peut consister à déposer une pellicule métallique à la surface d'une goutte de résine diélectrique transparente centrée sur l'axe O, fixée sur la paroi extérieure de la face de l'enceinte 12 sur laquelle est placée la goutte 11. On rabote le sommet de la goutte de résine au voisinage de l'axe O afin de laisser passer la lumière.

On peut faire croître la tension V de O volt à une tension maximale qui dépend des matériaux utilisés. Lorsque la tension maximale est atteinte, la goutte de liquide isolant 11 atteint une position limite (désignée par la référence B). Lorsque l'on fait varier V continûment entre O volt et sa valeur maximale, la goutte de liquide isolant 11 se déforme continûment de la position A à la position B. On notera que, la goutte 11 étant en un liquide isolant, il ne se produit pas de microgouttes à sa périphérie quand la tension est élevée, contrairement à ce qui se produirait si la goutte était en un liquide conducteur (voir l'article susmentionné de Vallet, Berge et Vovelle).

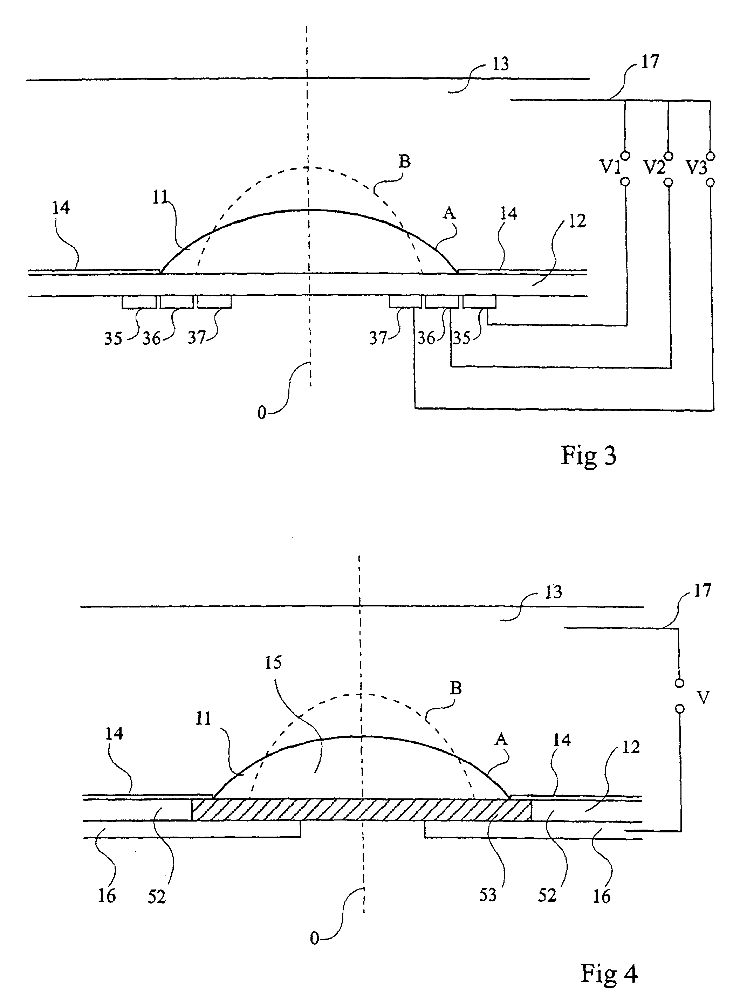

La figure 3 représente une vue en coupe simplifiée d'une lentille liquide à focale variable selon un troisième mode de réalisation de la présente invention. Les éléments tels que la goutte 11, l'axe O, l'enceinte 12, le liquide conducteur 13, le traitement de surface 14, la zone de contact 15 et l'électrode 17 sont les mêmes que dans le mode de réalisation décrit en figure 1. Les positions A et B correspondent également à la position de repos de la goutte 11 et à la position limite de la goutte 11 respectivement.

Dans ce troisième mode de réalisation, on dispose sur la face extérieure de la paroi de l'enceinte 12 un ensemble de trois électrodes circulaires concentriques isolées entre elles 35, 36 et 37 d'axe O. Un potentiel peut être appliqué entre chacune des électrodes 35, 36 et 37 et l'électrode 17, on a indiqué à titre d'exemple des tensions V1, V2 et V3, dont chacune peut varier. Les tensions sont choisies à tout instant de valeur décroissante vers l'axe O pour que le champ électrique généré par la mise sous tension des électrodes 35, 36, 37 décroisse radialement vers le centre de la zone 15. Lorsqu'on fait varier continûment les tensions V1, V2 et V3 entre O volt et leur valeur maximale, la goutte de liquide isolant 11 se déforme continûment entre sa position de repos A et sa position limite B.

Selon une variante de ce troisième mode de réalisation, chaque électrode 35, 36 et 37 peut être reliée par un commutateur soit à une même source de tension V soit à la masse. Pour une tension V constante, on fait alors varier la forme de la goutte 11 en faisant varier le nombre d'électrodes sous tension. Dans ce cas la variation de focale est discrète et non continue. On ne peut ainsi obtenir que certaines focales prédéterminées pour la lentille formée par la goutte 11, mais on bénéficie alors d'une commande en tension relativement simple à mettre en oeuvre.

La figure 4 représente une vue en coupe simplifiée d'une lentille liquide à focale variable selon un quatrième mode de réalisation de la présente invention. Les éléments tels que la goutte 11, l'axe O, le liquide conducteur 13, le traitement de surface 14, la zone de contact 15 et les électrodes 16 et 17 sont les mêmes que dans le mode de réalisation décrit en figure 1. Les positions A et B correspondent également à la position de repos de la goutte 11 et à la position limite de la goutte 11 respectivement.

Dans ce quatrième mode de réalisation, la paroi de l'enceinte diélectrique 52 sur laquelle est posée la goutte de liquide isolant 11 comprend une zone diélectrique circulaire 53, laissant passer la lumière autour de l'axe O. La zone 53 présente une faible mouillabilité vis-à-vis du liquide conducteur 13 en l'absence d'un traitement de surface 14. La zone 53 a été traitée de telle manière que sa constante diélectrique varie radialement et continûment vers l'axe O, et que le champ électrique généré par la tension V présente un gradient décroissant radialement vers l'axe O sur la zone de contact 15. Lorsqu'on fait varier continûment la tension V entre O volt et sa valeur maximale, la goutte de liquide isolant 11 se déforme continûment entre sa position de repos A et sa position limite B.

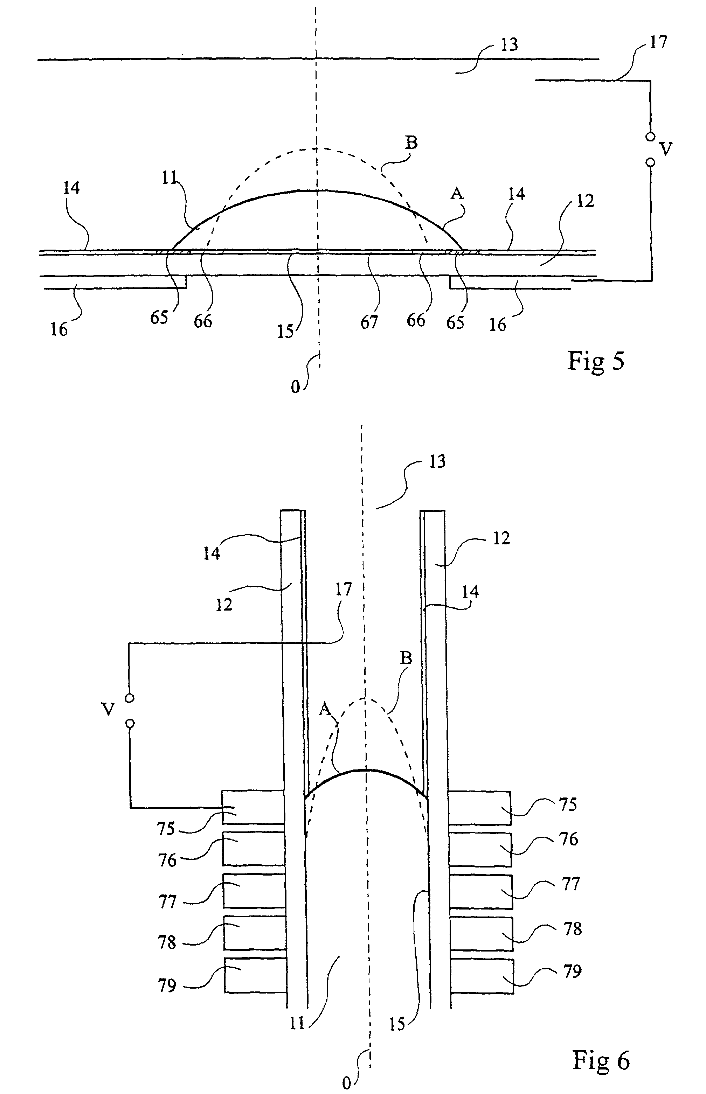

La figure 5 représente une vue en coupe simplifiée d'une lentille liquide à focale variable selon un cinquième mode de réalisation de la présente invention. Les éléments tels que la goutte 11, l'axe O, l'enceinte diélectrique 12, le liquide conducteur 13, la zone de contact 15 et les électrodes 16 et 17 sont les mêmes que dans le mode de réalisation décrit en figure 1. Les positions A et B correspondent également à la position de repos de la goutte 11 et à la position limite de la goutte 11 respectivement.

Dans ce cinquième mode de réalisation, la surface de la paroi de l'enceinte diélectrique 12 sur laquelle est posée la goutte de liquide isolant 11 a été traitée sur différentes zones 14, 65, 66 et 67 afin que la mouillabilité des zones 14, 65, 66, 67 vis-à-vis du liquide conducteur 13 décroisse radialement vers l'axe O. Un potentiel V peut être appliqué entre l'électrode 16 et l'électrode 17. Le champ électrique généré par la tension V accroît la mouillabilité des zones 14, 65, 66 et 67 mais conserve le gradient initial de mouillabilité. Lorsque la tension V évolue entre O V et sa valeur maximale, la forme de la goutte de liquide isolant 11 évolue continûment entre sa position de repos A et sa position limite B.

La figure 6 représente une vue en coupe simplifiée d'un autre mode de réalisation de la présente invention dans lequel un liquide isolant 11 occupe la partie inférieure de l'enceinte diélectrique cylindrique et est recouvert d'un liquide conducteur 13. L'enceinte est désignée par la référence 12. Les matériaux composant les éléments 11, 12 et 13 sont les mêmes que dans les modes de réalisation précédents.

Un traitement de surface 14 assurant une forte mouillabilité de la paroi interne de l'enceinte 12 vis-à-vis du liquide conducteur 13 est réalisé au-dessus de la zone de contact 15 entre le liquide 11 et la paroi interne de l'enceinte 12. Le traitement de surface 14 permet de conserver le positionnement du liquide 11, pour éviter que ce liquide ne s'étale au-delà de la surface de contact. Pour simplifier la description on ne considérera que la partie supérieure du liquide 11, que l'on appellera, comme dans les modes de réalisation précédents "goutte 11". Lorsque le système est au repos, la goutte de liquide isolant 11 prend naturellement la forme désignée par la référence A. On appelle O l'axe de l'enceinte 12. Au repos, la goutte de liquide isolant 11 est centrée sur l'axe O qui constitue l'axe optique du dispositif. Plusieurs électrodes 75, 76, 77, 78, 79 sont disposées autour de la paroi extérieure de l'enceinte diélectrique 12 au voisinage de la zone de contact 15. Les électrodes 75, 76, 77, 78, 79 sont isolées entre elles et on établit une tension V entre l'électrode 75 et une électrode 17 en contact avec le liquide conducteur 13. Les électrodes 76, 77, 78, 79 sont polarisées par influence capacitive lorsqu'on établit la tension V. Au niveau de la paroi 12, le champ électrique créé par la tension V décroît selon un gradient longitudinal depuis l'électrode 75 vers l'électrode 79. Lorsque la tension V augmente, le liquide conducteur 13 se déplace et déforme la goutte de liquide isolant 11. On obtient ainsi une variation de la focale de la lentille. Le gradient susmentionné de champ électrique assure que la goutte présente en permanence une symétrie radiale par rapport à l'axe O. Lorsque la tension V évolue entre 0 volt et sa valeur maximale, la goutte de liquide isolant 11 évolue continûment entre sa position de repos A et sa position maximale B.

L'homme de l'art pourra combiner les caractéristiques apparaissant dans les divers modes de réalisation de l'invention décrits ci-dessus.

De plus, la présente invention est susceptible de diverses variantes qui apparaîtront à l'homme de l'art.

La surface de l'enceinte diélectrique 12 de la figure 1 peut être concave ou convexe, afin d'obtenir une dioptrie particulière du dispositif au repos.

La zone de contact entre la goutte de liquide isolant et l'enceinte diélectrique peut être traitée pour présenter une forte mouillabilité vis-à-vis du liquide isolant, afin de faciliter le positionnement de la goutte de liquide isolant.

Dans le cas d'une enceinte diélectrique présentant naturellement une forte mouillabilité vis à vis du liquide conducteur, la zone de contact peut être réalisée par un traitement de surface destiné à lui donner une faible mouillabilité vis à vis du liquide conducteur.

Le traitement de surface 14 peut consister en un dépôt ou un collage d'un film d'un matériau présentant une forte mouillabilité vis-à-vis du liquide conducteur 13.

L'électrode 16 de la figure 1 peut être remplacée par un liquide conducteur en contact avec la face extérieure de l'enceinte 12, la tension V étant alors établie entre ce liquide conducteur et le liquide 13.

On pourra réaliser un dispositif comportant un réseau formé de groupes de trois lentilles à focale variable, commandées séparément, colorées en rouge, vert, bleu, fonctionnant par exemple en tout ou rien, permettant de laisser passer ou d'arrêter la lumière provenant d'une source unique de lumière blanche, formant ainsi un écran couleur lumineux pouvant être de très grande taille et de coût modéré.

On pourra réaliser un dispositif dans lequel les moyens de centrage susmentionnés ne sont plus utilisés pour maintenir la goutte 11 circulaire tout au long de sa déformation, mais au contraire pour la faire passer d'une forme de repos, déterminée par exemple par la forme du traitement de surface 14, à une forme en fonctionnement, déterminée par exemple par le contour de l'électrode 16. On pourra ainsi créer une lentille cylindrique à focale variable en utilisant un traitement de surface 14 de forme rectangulaire et des électrodes de centrage 16 de contour rectangulaire.

On pourra appliquer la présente invention à un dispositif à cheval sur plus d'une paroi de l'enceinte 12, la goutte 11 étant disposée par exemple dans un angle ou un coin de l'enceinte 12. Dans cette variante, bien sur, une électrode serait disposée sur la face arrière de chaque paroi en contact avec la goutte 11, au niveau de la zone de contact. Une telle variante permettrait par exemple de réaliser un prisme à déflexion variable.

A titre d'exemple on pourra utiliser comme liquide conducteur 13 de l'eau chargée en sels (minéraux ou autres) ou tout liquide, organique ou non, qui soit conducteur ou rendu tel par ajout de composés ioniques. Comme liquide isolant 11 on pourra utiliser de l'huile, un alcane ou mélange d'alcanes, éventuellement halogénés, ou tout autre liquide isolant et non miscible avec le liquide conducteur 13. L'enceinte 12 peut être composée d'une plaque de verre silanisée ou recouverte d'une fine couche de polymère fluoré ou d'une superposition de polymère fluoré, de résine époxy, de polyéthylène.

On utilisera de préférence comme tension V une tension alternative, afin d'éviter l'accumulation de charges électriques dans l'épaisseur du matériau 12, à partir de la surface sur laquelle est posée la goutte 11.

Dans l'exemple d'application de la figure 1, la goutte 11 a un diamètre au repos d'environ 6 mm. Le liquide conducteur 13 et le liquide isolant de la goutte 12 étant sensiblement de même densité, la goutte 12 a la forme d'une calotte sphérique. Lorsqu'elle est au repos (position A), le bord de la goutte 11 fait un angle d'environ 45 degrés avec la surface de l'enceinte 12. Dans sa position limite (position B), le bord de la goutte 11 fait un angle d'environ 90 degrés avec la surface de l'enceinte 12. Le dispositif décrit, utilisant comme liquide conducteur 13 de l'eau salée d'indice optique 1,35 et comme liquide isolant de la goutte 11 de l'huile d'indice optique 1,45, permet d'obtenir environ 40 dioptries de variation de focale pour une tension appliquée de 250 V et une puissance électrique dissipée de quelques mW. La fréquence de la tension alternative est dans ce cas comprise entre 50 et 10 000 Hz, sa période étant nettement inférieure au temps de réponse du système d'environ quelques centièmes de seconde.

La lentille à focale variable selon la présente invention peut avoir une taille comprise entre quelques dizaines de µm et quelques dizaines de mm et peut notamment être appliquée au domaine des systèmes optoélectroniques ou de l'endoscopie.

## Revendications

1. Lentille à focale variable comprenant une enceinte (12) remplie d'un premier liquide (13), une goutte d'un deuxième liquide (11) étant disposée au repos sur une zone d'une première face d'une paroi isolante de l'enceinte, les premier et deuxième liquides étant non miscibles, d'indices optiques différents et sensiblement de même densité, **caractérisée en ce que** :
le premier liquide est conducteur ;
le deuxième liquide est isolant ; et **en ce qu'**elle comprend :
des moyens pour appliquer une tension électrique entre le liquide conducteur et une électrode (16 ; 26 ; 35-37 ; 75-79) disposée sur la deuxième face de ladite paroi ; et
des moyens de centrage pour maintenir le centrage et contrôler la forme du bord de la goutte tandis qu'une tension est appliquée.

2. Lentille à focale variable selon la revendication 1, dans laquelle les moyens de centrage permettent de maintenir continûment le centrage de la goutte et de contrôler continûment la forme du bord de la goutte tandis qu'une tension variable est appliquée par lesdits moyens pour appliquer une tension électrique.

3. Lentille à focale variable selon la revendication 2, dans laquelle la première face est sensiblement plane, la zone de contact (15) est circulaire et centrée sur un axe (O) perpendiculaire à la première face.

4. Lentille à focale variable selon la revendication 3, dans laquelle les moyens de centrage correspondent à un épaississement progressif de la deuxième face de la paroi de l'enceinte vers ledit axe, ladite électrode (26) étant plaquée sur ladite deuxième face.

5. Lentille à focale variable selon la revendication 3, dans laquelle les moyens de centrage correspondent à une décroissance radiale de la mouillabilité vis-à-vis du premier liquide (13), vers le centre de ladite zone de contact (15) avec le deuxième liquide.

6. Lentille à focale variable selon la revendication 3, dans laquelle les moyens de centrage correspondent à une gradation radiale de la constante diélectrique de ladite paroi de l'enceinte (53) au niveau de ladite zone de contact (15) avec le deuxième liquide.

7. Lentille à focale variable selon la revendication 1, dans laquelle la première face est sensiblement plane, la zone de contact (15) est circulaire et centrée sur un axe (O) perpendiculaire à la première face, et où les moyens de centrage sont constitués d'une électrode composée d'une ou plusieurs bandes circulaires concentriques (35-37) isolées entre elles, centrées sur ledit axe, les bandes circulaires étant alimentées par des sources de tension distinctes de valeur décroissante vers ledit axe.

8. Lentille à focale variable selon la revendication 1, dans laquelle l'enceinte est cylindrique, la première face est la face intérieure de l'enceinte, la zone de contact avec le deuxième liquide correspond à une section cylindrique de l'enceinte, les moyens de centrage sont constitués d'une ou plusieurs électrodes cylindriques de même diamètre, isolées entre elles, disposées côte à côte contre la face extérieure de l'enceinte au niveau de la frontière de ladite zone de contact, les électrodes étant alimentées par des tensions différentes de valeur décroissante vers le milieu de ladite zone de contact.

9. Lentille à focale variable selon la revendication 1, dans laquelle la première face est sensiblement plane, la zone de contact (15) est rectangulaire et symétrique par rapport à un axe (O) perpendiculaire à la première face et les moyens de centrage sont constitués d'une électrode composée d'une ou plusieurs bandes rectangulaires concentriques isolées entre elles, symétriques par rapport audit axe (O), les bandes rectangulaires étant alimentées par des sources de tension distinctes de valeur décroissante vers ledit axe.

10. Lentille à focale variable selon la revendication 1 dans laquelle ladite paroi est composée de deux plans non parallèles et dans laquelle ladite zone se trouve à cheval sur lesdits deux plans.

## Claims

1. A variable focus lens comprising a chamber (12) filled with a first liquid (13), a drop of a second liquid (11) being disposed at rest on a region of a first surface of an insulating wall of the chamber, the first and second liquids being non miscible, of different optical indexes and of substantially same density, **characterized in that**:
the first liquid is conductive;
the second liquid is insulating;
**in that** it comprises:
means for applying a voltage between the conductor liquid and an electrode (16; 26; 35-37; 75-79) placed on the second surface of said wall; and
centering means for maintaining the centering of the edge of the drop while the voltage is applied and for controlling the shape thereof.

2. The variable focus lens according to claim 1, in which the centering means allows a continuous maintaining of the centering of the drop and a continuous control of the shape of the edge of the drop while a varying voltage is applied by said means for applying a voltage.

3. The variable focus lens according to claim 2, in which the first surface is substantially flat, the contact region (15) is circular and centered about an axis (O) which is perpendicular to the first surface.

4. The variable focus lens according to claim 3, in which the centering means corresponds to a progressive thickening of the second surface of the wall of the chamber towards said axis, said electrode (26) being applied against said second surface.

5. The variable focus lens according to claim 3, in which the centering means corresponds to a radial decrease of the wetting with respect to the first liquid (13), towards the center of said contact region (15) with the second liquid.

6. The variable focus lens according to claim 3, in which the centering means corresponds to a radial gradient of the dielectric constant of said wall of the chamber (53) at the level of said contact region (15) with the second liquid.

7. The variable focus lens according to claim 1, in which the first surface is substantially flat, the contact region (15) is circular and centered about an axis (O) perpendicular to the first surface, and wherein the centering means comprises an electrode formed of one or several circular concentric strips (35-37) insulated from each other, centered about said axis, the circular strips being supplied by distinct voltage sources of values decreasing towards said axis.

8. The variable focus lens according to claim 1, in which the chamber is cylindrical, the first surface is the internal surface of the chamber, the contact region with the second liquid corresponds to a cylindrical section of the chamber, the centering means is comprised of one or several cylindrical electrodes of same diameter, insulated from each other, placed side by side against the external surface of the chamber at the level of the border of said contact region, the electrodes being supplied by different voltages of values decreasing towards the center of said contact region.

9. The variable focus lens according to claim 1, in which the first surface is substantially flat, the contact region (15) is rectangular and symmetric with respect to an axis (O) perpendicular to the first surface and the centering means is comprised of an electrode formed of one or several rectangular concentric strips insulated from each other, symmetric with respect to said axis (O), the rectangular strips being supplied by distinct voltage sources of decreasing values towards said axis.

10. The variable focus lens according to claim 1, in which said wall is comprised of two non parallel planes and in which said region bridges said two planes.

## Patentansprüche

1. Verstellbare Fokuslinse mit einer Kammer (12), die mit einer ersten Flüssigkeit (13) gefüllt ist, wobei ein Tropfen einer zweiten Flüssigkeit (11) in einem Bereich einer ersten Oberfläche einer isolierenden Wand der Kammer in Ruhe angeordnet ist, wobei die erste und die zweite Flüssigkeit nicht mischbar sind und unterschiedliche optische Indizes sowie im wesentlichen dieselbe Dichte aufweisen, **dadurch gekennzeichnet, daß** die erste Flüssigkeit leitend ist; daß die zweite Flüssigkeit isolierend ist; und daß Mittel zum Anlegen einer Spannung zwischen der leitenden Flüssigkeit und einer Elektrode (16, 26, 35-37, 75-79), die auf der zweiten Oberfläche der Wand angeordnet ist, und Zentriermittel zum Aufrechterhalten der Zentrierung des Tropfenrandes bei angelegter Spannung sowie zum Steuern der Form des Tropfenrandes vorgesehen sind.

2. Fokuslinse nach Anspruch 1, **dadurch gekennzeichnet, daß** die Zentriermittel eine fortdauernde Aufrechterhaltung der Zentrierung des Tropfens und eine fortdauernde Überwachung der Form des Tropfenrandes erlauben, während eine variierende Spannung mit Hilfe der Mittel zum Anlegen einer Spannung angelegt wird.

3. Fokuslinse nach Anspruch 2, **dadurch gekennzeichnet, daß** die erste Oberfläche im wesentlichen flach ist, wobei der Kontaktbereich (15) kreisrund und um eine Achse (O) zentriert ist, welche senkrecht zu der ersten Oberfläche steht.

4. Fokuslinse nach Anspruch 3, **dadurch gekennzeichnet, daß** die Zentriermittel einer zunehmenden Verdickung der zweiten Oberfläche der Wand der Kammer in Richtung der Achse entsprechen, wobei die Elektrode (26) an die zweite Oberfläche angelegt ist.

5. Fokuslinse nach Anspruch 3, **dadurch gekennzeichnet, daß** die Zentriermittel einer radialen Verminderung der Benetzung durch die zweite Flüssigkeit bezüglich der ersten Flüssigkeit (13) in Richtung auf die Mitte des Kontaktbereichs (15) entsprechen.

6. Vorrichtung nach Anspruch 3, **dadurch gekennzeichnet, daß** die Zentriermittel einen radialen Gradienten der dielektrischen Konstante der Wand der Kammer (53) auf dem Niveau des Kontaktbereichs (15) mit der zweiten Flüssigkeit entsprechen.

7. Fokuslinse nach Anspruch 1, **dadurch gekennzeichnet, daß** die erste Oberfläche im wesentlichen flach ist, der Kontaktbereich (15) kreisrund und zentriert um eine Achse (O) ist, die senkrecht zur ersten Oberfläche ist, und daß die Zentriermittel eine Elektrode umfassen, die einen oder mehrere kreisrunde, konzentrische, voneinander isolierte Streifen (35-37) aufweist, die um die Achse zentriert sind, wobei die kreisrunden Streifen von gesonderten Spannungsquellen mit Spannungswerten gespeist sind, die in Richtung der Achse abnehmen.

8. Fokuslinse nach Anspruch 1, **dadurch gekennzeichnet, daß** die Kammer zylindrisch ist, die erste Oberfläche die innere Oberfläche der Kammer ist, der Kontaktbereich mit der zweiten Flüssigkeit einem zylindrischen Abschnitt der Kammer entspricht, die Zentriermittel eine oder mehrere zylindrische, voneinander isolierte Elektroden gleichen Durchmessers umfassen, die Seite an Seite an der äußeren Oberfläche der Kammer auf einem Niveau der Grenze des Kontaktbereichs angeordnet sind, wobei die Elektroden von unterschiedlichen Spannungen mit Spannungswerten gespeist sind, die in Richtung zur Mitte des Kontaktbereichs abnehmen.

9. Fokuslinse nach Anspruch 1, **dadurch gekennzeichnet, daß** die erste Oberfläche im wesentlichen flach ist, der Kontaktbereich (15) rechteckig und bezüglich einer Achse (O) symmetrisch ist, die senkrecht zur ersten Oberfläche steht, und daß die Zentriermittel eine Elektrode umfassen, die von einem oder mehreren rechteckigen, konzentrischen, voneinander isolierten Streifen gebildet ist, die bezüglich der Achse (O) symmetrisch sind, wobei die rechteckigen Streifen von gesonderten Spannungsquellen gespeist sind, die in Richtung der Achse abnehmende Spannungswerte aufweisen.

10. Fokuslinse nach Anspruch 1, **dadurch gekennzeichnet, daß** die Wand zwei nicht-parallele Ebenen aufweist, wobei der Bereich die zwei Ebenen überbrückt.
